# EUROPEAN PATENT APPLICATION

(11) **EP 2 060 227 A1**
(43) Date of publication of application: **20.05.2009**
(21) Application number: 08016392.6
(22) Date of filing: 17.09.2008
(51) Int. Cl.: A61B 5/00

(54) **Multimodal imaging system for tissue imaging**

(30) Priority: 15.11.2007 US 940463
(71) Applicant: Carestream Health, Inc., Rochester, NY 14608-1733 (US)
(72) Inventor: Liang, Rongguang, Penfield New York 14526 (US)
(74) Representative: Wagner, Karl H.

(57) **Abstract**

An apparatus for obtaining area images and optical coherence tomography (OCT) image of a tissue comprises an image sensor, a first illumination means providing broadband polarized polychromatic light, a second illumination means providing narrow-band ultraviolet light, a third illumination means providing Near-Infrared light, an optical coherence tomography (OCT) imaging apparatus, one or more polarization elements, and an optical filter. An image processor identifies a region of interest according to area image, which can be a polarized reflectance image, a fluorescence light image, or both for the OCT imaging apparatus to obtain an OCT image over the region of interest.

## Description

### CROSS REFERENCE TO RELATED APPLICATIONS

Reference is made to commonly-assigned co-pending U.S. Application Serial Number 11/262,869, filed October 31, 2005, entitled METHOD FOR DETECTION OF CARIES, by Wong et al.; U.S. Application Serial Number 11/408,360, filed April 21, 2006, entitled OPTICAL DETECTION OF DENTAL CARIES by Wong et al.; U.S. Application Serial No. 11/530, 987, filed September 12, 2006, entitled APPARATUS FOR CARIES DETECTION, by Liang et al.; U.S. Application Serial No. 11/530,913. filed September 12, 2006, entitled LOW COHERENCE DENTAL OCT IMAGING, by Liang et al.; and U.S. Application Serial No. 11/561,971 filed November 21, 2006, entitled Apparatus for Dental OCT Imaging, by Liang et al, the disclosures of which are incorporated herein.

### FIELD OF THE INVENTION

This invention generally relates to methods and apparatus for tissue imaging using polarized visible light, ultraviolet light, polarized Near-Infrared light, and optical coherence tomography (OCT) imaging.

### BACKGROUND OF THE INVENTION

The common method for examining the tissue and detecting pre-malignant lesions includes visual inspection, followed by biopsy of any suspicious lesions. However, biopsy is not only invasive and time-consuming; but also, a practitioner or operator may find it difficult to determine the optimal, i.e., most dysplastic, location for biopsy, leading to repeated biopsies and the risk of underdiagnosis. Therefore, there is a need for techniques that can distinguish between different types of lesions reliably and non-invasively.

Recently, optical methods, such as reflectance imaging, fluorescence imaging, and Optical Coherence Tomography (OCT) imaging, have been developed for early detection of biochemical and structural changes in tissue. Reflectance imaging and fluorescence imaging are defined as area imaging in this application since both of them obtain images of an area. It has been shown that light scattering properties differ between tumor and healthy tissue. In particular, reflectance of light from the illuminated area can be at measurably different levels for normal versus abnormal areas. This change in reflectance may not be sufficiently pronounced to be of diagnostic value when considered by itself, since the change in reflectance of light is very slight, although detectable. In broadest optical parlance, reflectance generally denotes the sum total of both specular reflectance and scattered reflectance. Specular reflection is that component of the light that is reflected by the tissue surface and the superficial layer of the tissue at the same angle as the incident angle. For some biomedical applications, the specular component of reflectance is of no interest and is, instead, generally detrimental to obtaining an image or measurement from a sample. The component of reflectance that is of interest for the present application is from scattered light inside the tissue only. With specular reflection removed, the scattered light can provide information of the subsurface structure of the tissue.. For some biomedical applications, such as the birefringence measurement, images with both polarization states must be captured. Therefore, it is difficult to remove the specular reflection only without filtering out the scattering signal with the same polarization state as the specular reflection.

Autofluorescence of tissues is produced by fluorophores in cells with a suitable light excitation, and its profile changes in the disease state since the concentration of fluorophores is altered. While autofluorescence provides a sensitive method to detect early tissue transformation, significant challenges include the low signal-to-noise ratio (SNR), difficulty in quantifying data, and limited tissue penetration.

OCT is a high-resolution optical technique that enables imaging of near-surface abnormalities in complex tissues. The presence of disease changes the light scattering and absorption properties of the tissue, due to changes in blood concentration, nuclear size distribution, collagen content and epithelial thickness. While OCT solutions can provide very detailed image of structure beneath the surface of a tissue, OCT imaging itself can be time-consuming and its field of view is limited. OCT images would be most valuable if obtained within one or more local regions of interest, rather than obtained over widespread areas. That is, once a user identifies a specific area of interest, then OCT imaging could be directed to that particular area only.

In recent years, there have been a lot of efforts on developing new optical technologies in tissue imaging and characterization. U. S. Patent No. 6,177,984 assigned to Jacques presented a device to image the superficial biological tissue layer using polarized light. This device takes a set of measurements using a broad illumination beam of light circularly polarized or linearly polarized at different angles for tissue characterization. Boppart et al presented a method combining white light endoscopic imaging and OCT imaging methods for tissue imaging in a paper entitled "Forward imaging instruments for Optical Coherence Tomography" Optics Letter, Vol. 22, 1618-1620 (1997). However, specular reflection in white light imaging presents an issue when identifying the suspicious region. U.S. Patent No. 6,507,747 (Gowda et al.) described an optical imaging probe that includes both a spectroscopic imaging probe element and an OCT imaging probe element. This device uses a fluorescence image to guide an OCT scan. However, while fluorescence is sensitive to very early tissue transformation, it is also sensitive to bacteria, food and plaque as well. Fluorescence image only may lead to unnecessary OCT scan.

While methods and apparatus for combined area imaging and OCT scanning have been proposed, there remains considerable room for improvement. Optical apparatus and methods disclosed in the cited patents, co-pending applications and paper fall short of what is needed for tissue imaging that combines these imaging functions with suitable image quality and is yet compact and easy to use.

Thus, it can be seen that there is a need for a tissue imaging apparatus that provides both area and OCT imaging in a compact package.

### SUMMARY OF THE INVENTION

Briefly, according to one aspect of the present invention an apparatus for obtaining area images and an OCT image of a tissue comprises an image sensor, a first illumination means providing broadband polarized polychromatic light, a second illumination means providing narrow-band ultraviolet light, a third illumination means providing polarized near-infrared (NIR) light, an OCT imaging apparatus, one or more polarization elements, and an optical filter. An image processor identifies a region of interest according to area image, which can be a polarized reflectance image, a fluorescence light image, or both for the OCT imaging apparatus to obtain an OCT image over the region of interest.

According to another aspect of the invention, a method for obtaining images of a tissue comprises steps of directing broadband polarized polychromatic light onto the tissue surface for obtaining a polarized reflectance image of the tissue; directing narrow-band ultraviolet light onto the tissue surface for obtaining a fluorescence image of the tissue; directing near infrared light onto the tissue surface for obtaining reflectance images with orthogonal polarization states; displaying the polarized reflectance images and fluorescence image of the tissue on a display unit; identifying the region of interest on the tissue from the reflectance and fluorescence images; scanning the identified area and obtaining an OCT image; and storing at least one parameter of the region of interest and OCT scanning.

The use of image analysis logic for determining, from both polarized reflectance image and fluorescence image, the region of interest for OCT scanning is a feature of the present invention.

The method of the present invention to register area images and OCT image is another feature of the present invention.

The apparatus of the present invention to obtain the tissue properties of superficial surface is further a feature of the present invention.

The method of the present invention is advantaged over earlier area imaging methods in that it removes the specular reflection in reflectance imaging.

The method of the present invention is also advantaged over earlier area imaging methods in that it combines polarized reflectance image and fluorescence image to obtain contrast enhanced area image.

The method of the present invention is further advantaged over earlier methods for OCT imaging in that it uses polarized reflectance image and fluorescence image to detect a region of interest to guide OCT imaging for detailed assessment over that region.

These and other objects, features, and advantages of the present invention will become apparent to those skilled in the art upon a reading of the following detailed description when taken in conjunction with the drawings wherein there is shown and described an illustrative embodiment of the invention.

### BRIEF DESCRIPTION OF THE DRAWINGS

While the specification concludes with claims particularly pointing out and distinctly claiming the subject matter of the present invention, it is believed that the invention will be better understood from the following description when taken in conjunction with the accompanying drawings, wherein:
FIG. 1a is a schematic block diagram of an imaging apparatus for tissue imaging providing both area imaging and OCT imaging;
FIG. 1b is a view taken along line b-b of FIG. 1a;
FIG. 1c is a diagram of different configurations of illumination means;
FIG. 1d is a schematic block diagram showing components of an OCT imaging system;
FIG. 2 is a diagram of the transmission curves of the band pass and long pass filter, as well as the spectrum of the light source;
FIG. 3 is a diagram of the region of interest identification;
FIG. 4 is a logic flow diagram of a sequence of operator steps that are used to obtain area images and OCT image;
FIG. 5a is a diagram of an alternative embodiment with two sensors;
FIG. 5b is a view taken along line b-b of FIG. 5a;
FIG. 6 is a diagram of another alternative embodiment with two sensors and a dichroic filter to combine area imaging and OCT imaging;
FIG. 7 is a diagram of an imaging apparatus with focus detection capability;
FIG. 8 is a diagram showing how focus detection method works;
FIG. 9 is a schematic block diagram of an imaging apparatus for tissue imaging using polarized light and OCT scanning in an alternate embodiment of the present invention.

### DETAILED DESCRIPTION OF THE INVENTION

The present description is directed in particular to elements forming part of, or cooperating more directly with, apparatus in accordance with the invention. It is to be understood that elements not specifically shown or described may take various forms well known to those skilled in the art.

The present invention combines area imaging capabilities, namely polarized reflectance imaging and fluorescence imaging, for identifying a region or regions of interest on the tissue surface with OCT imaging capabilities for obtaining detailed OCT scan data over a specified portion of the tissue corresponding to a portion of the region of interest.

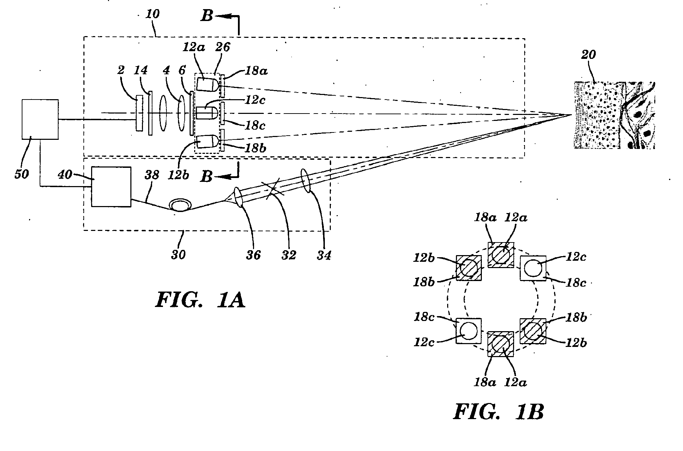

FIGS. 1a and 1b show an imaging apparatus comprising both an area imaging system 10 and an OCT imaging system 30 according to one embodiment. As part of imaging system 10, illumination means 12a, 12b and 12c provide uniform illumination on the tissue surface for area imaging. An imaging lens 4 images the surface of an area of tissue 20 onto an image sensor 2. Image sensor 2 can be a color charge-coupled device (CCD) or complimentary metal-oxide semiconductor (CMOS) image sensor.

The light sources in illumination means 12a-c can be ultraviolet, NIR or polychromatic. The light source for each illumination means emits light with different wavelengths for different imaging modes. For example, a light source in illumination means 12a emits broadband visible light (400nm - 700nm) for polarized reflectance imaging; or it can be a combination of the light sources with different spectrum, such as a combination of red, green and blue light emitting diodes (LEDs). A light source in illumination means 12b emits narrow band UV light (350nm - 450nm) to excite tissue fluorescence. A light source in illumination means 12c is an NIR light source for deep tissue imaging, having a wavelength longer than 700nm. Each polychromatic light source can have a corresponding polarizer, as shown by polarizers 18a, for polarized illumination. Each ultraviolet light source has a band-pass filter 18b to clean the spectrum. For NIR illumination, a polarizer 18c in front of the illumination means 12c is an option. It can be used when polarized illumination is needed. In this embodiment, polarizer 18a is placed in front of illumination means 12a to provide polarized light illumination on the tissue surface. A single broadband light source, together with spectral selection filters, can be used to replace the light sources in illumination means 12a, 12b, and 12c in current configuration. A broadband band-pass filter is necessary to provide broadband light illumination for reflectance image; and two narrow-band band-pass filters are used for fluorescence and NIR imaging. In certain situations, the single broadband light source and spectrum selection filter may not be able to be integrated into a handheld unit, a fiber or liquid light guide could be used to deliver the light to each illumination means 12a-c.

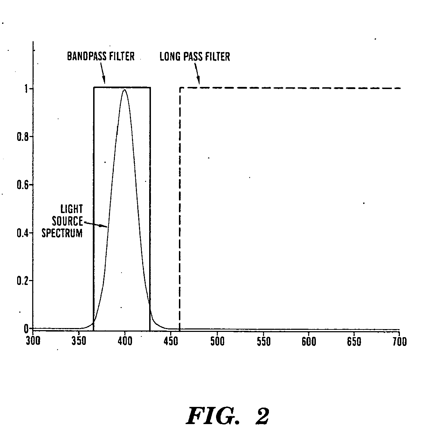

To remove the specular reflection from the tissue surface, an analyzer 14 is placed in the image path in front of the sensor 2. In this configuration, the ultraviolet light from illumination means 12b is not polarized, but band-pass filter 18b is effective to clean the spectrum of the ultraviolet light to minimize the cross talk in fluorescence image. In order to block the excitation light from illumination means 12b from reaching sensor 2 when taking fluorescence image, a long pass filter 6 is placed in front of the imaging lens 4. To block the excitation light efficiently and maintain the full spectrum of the visible light for reflectance imaging, the cut-on wavelength of long pass filter 6 should be greater than the cut-off wavelength of band-pass filter 18b, but smaller than the blue end of the visible spectrum, at 470nm. Figure 2 shows the transmission curves of the band-pass and long pass filters, as well as the spectrum of the light source. As indicated in FIG. 2, the cut-on wavelength of the long pass filter is greater than the cut-off wavelength of the band pass filter.

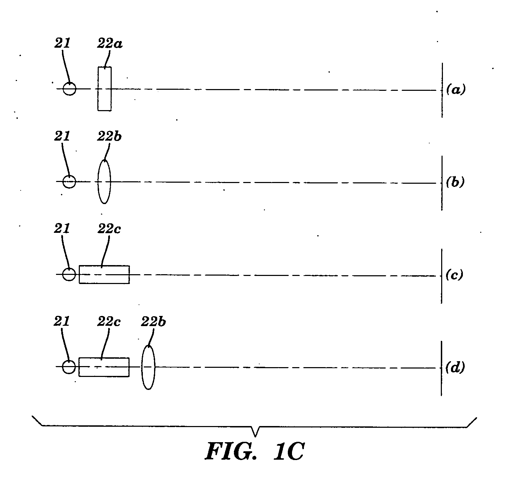

Illumination uniformity is important in tissue imaging as the intensity of the image is used to analyze the tissue property. If the illumination is not uniform, the analysis will be inaccurate, or misleading. In order to provide uniform illumination on tissue surface, each of illumination means 12a-12c may comprise at least one light source and one or more beam shaping optics. Each illumination means could have its own light source, or a single light source with spectral selection filter provides light with different illumination spectrum for each illumination. The light source could be any of light emitting diode (LED), laser, and broadband light source such as xenon arc lamp. FIG. 1c shows different configurations for illumination means useful in present invention. In these configurations, a light source 21 and beam shaping optics 22a-c are combined to shape the light for uniform illumination on the tissue surface. If the beam profile from the light source is uniform enough for illumination on the tissue surface, no beam shaping optics will be needed. Beam shaping optics can be a diffuser 22a as shown in FIG. 1c(a), a spherical or aspheric optical element 22b in FIG. 1c(b), a light pipe 22c in FIG. 1c(c), or a combination of light pipe 22c and imaging lens 22b in FIG. 1c(d). The path of illumination light might further include light guiding or light distributing structures so that the tissue surface is illuminated uniformly.

In OCT imaging system 30, NIR light from a sample arm fiber 38 is collimated by collimating lens 36, and then hits scanning element 32. Scanning element 32 could be polygon, galvanometer, or micro-electro-mechanical systems (MEMS) scanner. Together with scanning lens 34, scanning element 32 scans NIR light from the fiber 38 across the tissue surface for OCT imaging.

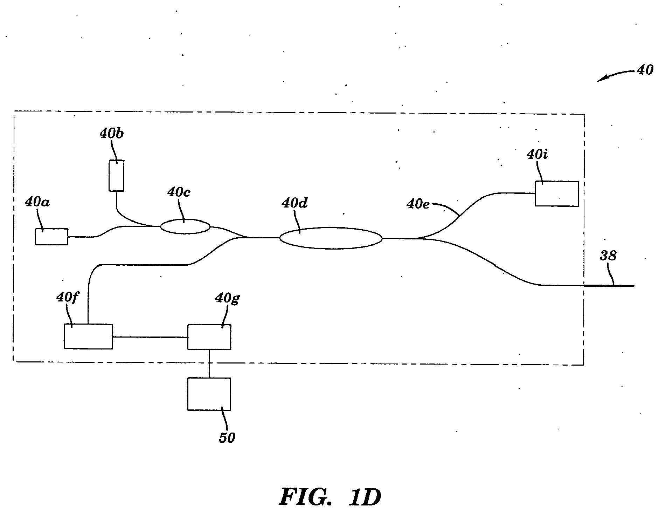

Figure 1d shows a diagram of the components of an example OCT engine 40, which can be a time-domain, a Fourier-domain system, or a polarization sensitive OCT system. Light provided by OCT light source 40a can be a continuos wave low coherence broadband light, and may be from a source such as a super-luminescent diode (SLD), diode-pumped solid-state crystal source, diode-pumped rare earth-doped fiber source, or tunable broadband light, for example. In one embodiment, NIR light is used, such as light having wavelengths near 1310 nm, for example. Usually OCT light source 40a has the wavelength in NIR, for example, at around 1310nm, in order to obtain enough depth inside the object under investigation. Alternatively, light source 40a can operate at around 850 nm. When working with a Fourier Domain instrument the OCT light source 40a can be a tunable light source. Optional visible light source 40b, at a different wavelength than light source 40a, aids in OCT scan visualization. This is useful to show where the OCT light is scanning on the tissue surface during line or area scans so that the practitioner can see where measurements actually are performed. Light source 40b can be a visible laser or laser diode, light emitting diode (LED), or other light source, for example centered on 650nm. A 2-to-1 coupler 40c combines the light from light sources 40a and 40b and sends the light to a 2 by 2 coupler 40d, which also acts as the active element of the interferometer. After passing coupler 40d, the light from light sources 40a and 40b separates into a reference arm optical fiber 40e and sample arm optical fiber 38. Light traveling down reference arm optical fiber 40e is incident upon the reference delay depth scanner 40i. The purpose of the reference delay depth scanner 40i is to change the path length of the reference arm of the interferometer relative to the sample arm. The reference delay depth scanner 40i includes a reflector (not shown), which causes the delayed light to travel back down reference arm optical fiber 40e. The light signals returned from reference and sample arms are recombined by 2 by 2 coupler 40d to form the interference signal. The interferometric signal is detected by detector and detection electronics 40f as a function of time. The detected signal is collected by a control logic processor and computer 50 after processing though signal processing electronics 40g, for example, low pass filter and logarithm of the envelope of the interference signal amplifier. The detector 40f can either be a balanced detector or a single ended photodetector. If a balanced detector is used there is usually an optical circulator added to the OCT engine 40 between elements 40c and 40d.

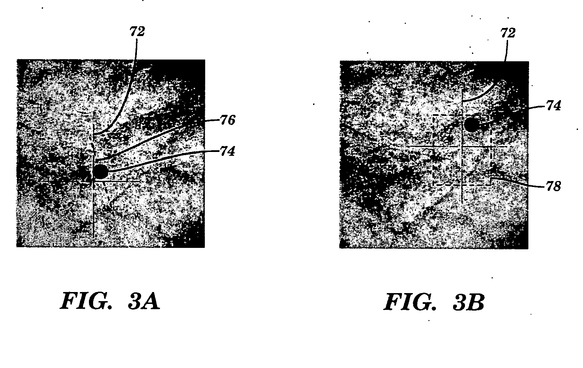

Figure 3 show how the operator specifies the location and region for OCT scanning. As shown in FIG. 3(a) and (b), a crosshairs 72, a light indicator 74, or other reference can be positioned suitably as various types of markers with respect to the tissue. Light indicator 74 can emanate from the OCT light source and could indicate the present location of the OCT scanning point on the tissue. Preferably the OCT scanning position would be centered on the scanning lens 34 so as to maximize the possible scanning area during OCT imaging procedure. Alternatively, the center of crosshairs 72 could indicate the center position of the OCT scanning area. When the OCT scan is a line scan as shown in Figure 3(a), a rotating thumbwheel (not illustrated) on the probe itself can be used to pivot line scan marker 76 relative to crosshairs 72, light indicator 74, or similar reference. Optionally, a mouse or joystick could be used by the operator or a touch screen interface could be employed for accepting the operator instruction. In one embodiment, an OCT volume image is simply defined by a fixed size rectangle, for example scan area 78 in Figure 3(b), which is centered with respect to the origin of the crosshairs 72. The rectangle can be changed in size and orientation according to operator instructions.

The operator can specify whether the scanning area requires a single line scan or a multiple-line volume scan, as well as the direction and density of measured points in the scan. When a volume image is selected for the scanning area, the density of adjacent scans is also selected. As an example, scan area 78 selected in Figure 3 (b) could be a 4 mm square region. Repetitive OCT line scans are performed on the tissue to form the volume scan. For example, the operator can elect to start in the top left corner of the region, to scan left to right in a raster fashion, and to use a 25 micron step size in orthogonal direction. The operator can also select the scan depth if desired.

One of the features of the current invention is that it stores the area images and the data from OCT scan simultaneously, as well as the parameters for OCT scan, such as the coordinates and size of region of interest, scanning mode (line scan or area scan), scanning direction, and the scanning step size. For example, once the region of interest is selected and the OCT scan starts, the area imaging system could acquire and store polarized reflectance and fluorescence images by turning on the light sources in illumination means 12a and 12b while OCT system is scanning the tissue surface. The system can also place the marker, such as a line 72 or a box 78, onto the saved area images so that the region of interest and OCT scan region are obvious to the users when the area images are displayed. This feature is very valuable for further image analysis. With OCT scanning parameters, OCT scans for each point in area images can be retrieved to analyze the tissue properties under the surface.

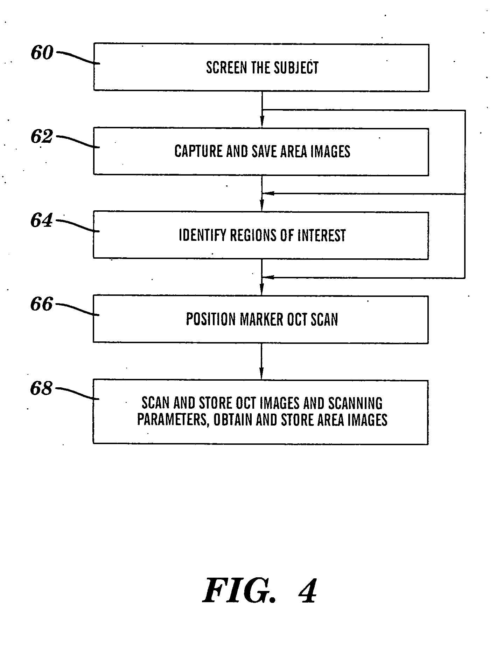

The flow diagram of Figure 4 shows how this system works. As the first step 60, the operator uses this device to screen the tissue with polarized white light on. The operator can also screen the tissue using fluorescence imaging at a video rate by turning ultraviolet light on and white light off. The operator can even obtain polarized reflectance image and fluorescence image alternatively at a video rate by switching white light and ultraviolet light on and off respectively. In next step 62, the operator captures and saves area images for further analysis. Alternately, the system may continuously (that is, repeatedly) perform this area imaging process, so that the operator continuously has a reference image displayed, enabling the operator to determine whether or not the probe is suitably positioned and the area image is in clear focus before proceeding to a later step. With area images, namely the polarized reflectance and fluorescence images, the operator or the image processing software can identify the region of interest in the following step 64.

The operator can also identify the region of interest when screening the subject without capturing and saving area images. Once the region of interest, such as a line or an area, is identified, at step 66 the operator positions the marker on the region of interest, saves the parameter of the marker, and then initiates the next step 68 - scan and store OCT images, as well as obtain and store area images. The operator can further skip steps 62, 64, and go to step 66 directly to position the marker for OCT scanning. After the OCT scanning region is identified, the OCT scans are obtained in step 68 of Figure 4. Typically the OCT displays are shown on the display screen in sequence as they are being generated.

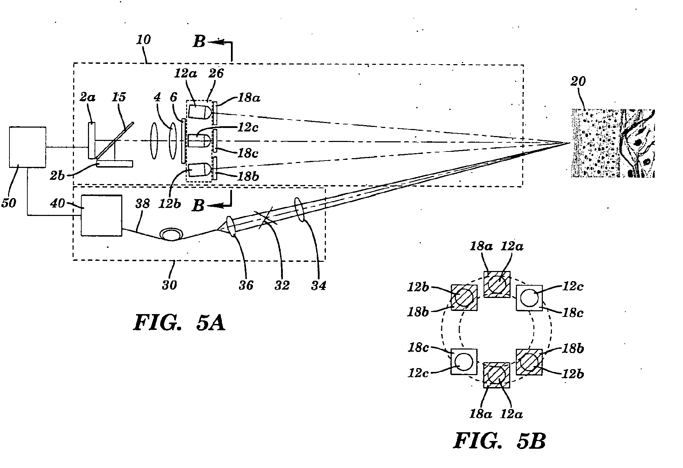

The polarized reflectance image under discussion so far is generated from the scattered light inside the tissue. It doesn't contain the information of the tissue surface and only contains little information of the superficial layer under the tissue surface. The reason is that the polarized light returned from the tissue surface and superficial layer has the same polarization state as the illumination beam, and is blocked by the analyzer 14 in order to remove the specular reflection. In certain situations, it is valuable to have images with both polarization states. For example, the two images can be further processed to gain the birefringent property of the tissue. If the polarization axes of the polarizer and analyzer used in present invention are electronically controllable, such as a liquid crystal element, a number of image pairs with orthogonal polarization states can be captured by changing the polarization directions of the illumination light and imaging light, and analysis for a complete polarization property of the tissue. Due to the tissue properties, the NIR light is preferred in this imaging mode since it penetrates deeper inside the tissue with less scattering and absorption. FIGS. 5a and 5b show a system that can obtain reflectance images with both polarization states with NIR light illumination provided by the third illumination means 12c, polarized reflectance image with white light illumination, fluorescence image and OCT images.

The difference between the embodiment of FIGS. 5a and 5b and that of FIG. 1 is that the embodiment in FIGS. 5a and 5b uses two sensors, 2a and 2b, and a polarization beam splitter 15 to replace the analyzer 14 in FIG. 1. The NIR light source in illumination means 12c provides uniform illumination on the tissue surface. The polarization beam splitter 15 separates the light returned from the tissue into two parts with different polarization states. One with the same polarization goes to one sensor said 2a, another with orthogonal polarization to sensor 2b. This embodiment can still obtain polarized reflectance image without specular reflection from sensor 2b. The polarized images from sensors 2a and 2b can be future processed to analyze the optical properties of the tissue surface and superficial layers for tissue with weak or no birefringence. For the tissue with strong birefringence, this difference is also an indicator of the degree of birefringence. The difference between the two images from the two sensors can also be normalized by the sum of the two images to cancel the interference from the superficial pigmentation for the measurement of the polarized light scattered from the superficial.

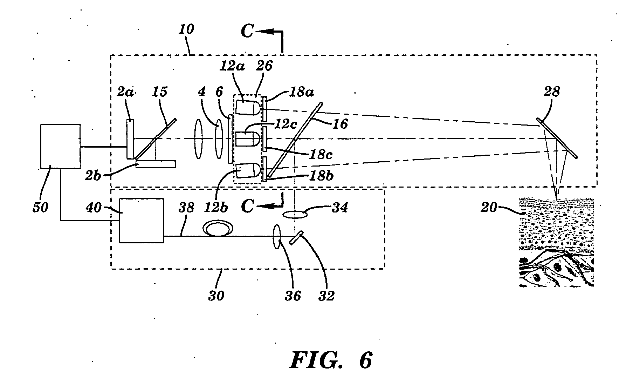

FIG. 6 shows another preferred embodiment of the present invention. In this configuration, a dichroic filter 16 is used to combine the area imaging and OCT imaging so that the optical axes of the both systems overlap after dichroic filter 16. Dichroic filter 16 in this embodiment transmits the light from the illumination means 12a-c, and reflects the light from the OCT imaging system. For example, it transmits the light with the wavelength less than 750nm, and reflects the light with the wavelength greater than 800nm. The system is more compact with the optical axes overlapping. Another component added in this embodiment is folding mirror 28. With this folding mirror, the probe has the flexibility to image the tissue in certain location, for example, the oral cavity.

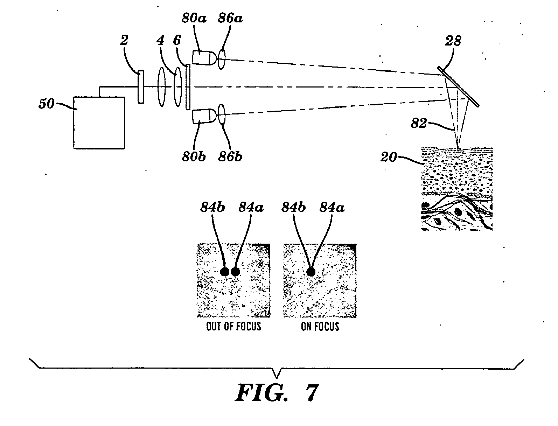

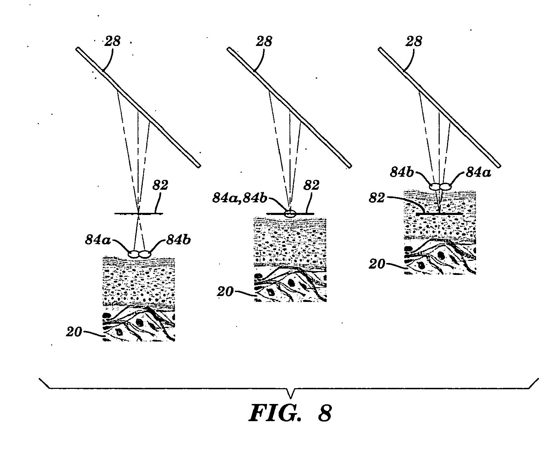

The apparatus of the present invention provides focus detection by imaging multiple light sources onto the tissue surface and aligning or overlapping the images formed from these light sources. FIG. 7 shows a dynamic focus detection embodiment using this method. First and second aiming light sources 80a and 80b and their imaging lenses 86a and 86b are arranged so that their images 84a and 84b overlap on the object plane 82 of the imaging lens 4. The imaging lenses 86a and 86b are not necessary if the light beams from the first and second aiming light sources 80a and 80b are collimated. In this case, images 84a and 84b represent the illumination spots on the tissue surface from the first and second aiming light sources. Object plane 82 is also the focal plane of the OCT scan lens 34. When the tissue surface is out of the focus, the two images 84a and 84b don't overlap as shown in Figure 7. The operator can move the imaging apparatus toward or away from the tissue surface until images 84a and 84b overlap. With this capability, the operator can always obtain the image at best focus. FIG. 8 shows, from a side view, how this overlap of images 84a and 84b works. An object plane 82 is indicated for imaging probe optics. At left, object plane 82 lies above tissue surface. At the right, object plane 82 lies below the surface of tissue. At center, object plane 82 is properly located on the surface of tissue 20 and images 84a and 84b overlap. Light sources 80a and 80b can be polychromatic light, or monochromatic light, such as LEDs or laser diodes with the wavelength longer than the cut off wavelength of the long pass filter 6. Other methods for focus detection can be implemented in the present invention too. For example, with predefined marker, only one light source, such as 80a and its corresponded imaging optics 86a, is necessary for focus detection.

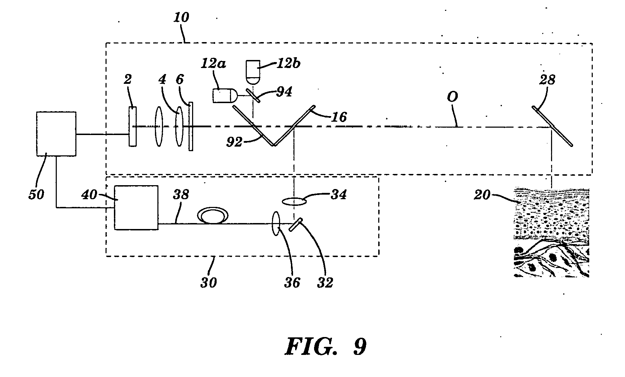

Various configurations of illumination means could be used, with various different embodiments employing only one polarization element. FIG. 9 shows such an embodiment. As part of a surface area imaging system, illumination means 12a provides illumination in the visible spectrum. An illumination combiner 94, such as a dichroic mirror, directs this visible light from illumination means 12a to a polarizing beam splitter 92, which directs light of the desired polarization state through a dichroic combiner 16 along optical axis O and toward a folding mirror 28 that directs the light toward a tissue 20. Illumination means 12b provides light outside the visible spectrum, such as UV light used to excite fluorescence from tissue 20. Light from illumination means 12b is directed through illumination combiner 94 to polarizing beam splitter 92 and along optical axis O. Image-bearing light returned from tissue 20 then travels back along optical axis O, through dichroic combiner 16 to polarizing beam splitter 92. Polarizing beam splitter 92 advantageously performs the functions of both the polarizer for illumination from illumination means 12a and 12b, and the analyzer for image bearing light, thus offering an efficient solution for polarization management. Tracing the path of illumination and image-bearing light shows how polarizing beam splitter 92 performs this function. Illumination from each light source is essentially unpolarized. In one embodiment, polarizing beam splitter 92 transmits P-polarization, and reflects S-polarization, directing this light to tissue 20. Polarizing beam splitter 92 treats the backscattered light in the same manner, transmitting the P-polarization and reflecting the S-polarization. The resulting P-polarized light can then be detected to form the surface area image at an imaging sensor 2. Because specular reflected light is S-polarized, polarizing beam splitter 92 effectively removes this specular reflective component from the light that reaches sensor 2. The optical path to sensor 2 has a lens 4, such as a compound lens as shown, and a long-pass filter 6 to block the light that is from illumination means 12b to excite fluorescence.

The invention has been described in detail with particular reference to certain preferred embodiments thereof, but it will be understood that variations and modifications can be effected within the scope of the invention as described above, and as noted in the appended claims, by a person of ordinary skill in the art without departing from the scope of the invention.

### PARTS LIST

- 2: image sensor
- 2a, 2b: image sensor
- 4: imaging lens
- 6: long pass filter
- 10: area imaging system
- 12a: illumination means for broadband visible light
- 12b: illumination means for narrow band ultraviolet light
- 12c: illumination means for NIR light
- 14: analyzer
- 15: polarization beam splitter
- 16: dichroic filter
- 18a: polarizer
- 18b: band-pass filter
- 18c: polarizer
- 20: tissue
- 21: light source
- 22a: diffuser
- 22b: spherical or aspherical optical element
- 22c: light pipe
- 28: folding mirror
- 30: OCT imaging system
- 32: scanning element
- 34: scanning lens
- 36: collimating lens
- 38: sample arm optical fiber
- 40: OCT engine
- 40a: OCT light source
- 40b: visible light source
- 40c: 2-to-1 coupler
- 40d: 2 by 2 coupler (interferometer)
- 40e: reference arm optical fiber
- 40f: detector and detection electronics
- 40g: signal processing electronics
- 50: computer and control system
- 60: screen the subject step
- 62: capture and save area images step
- 64: identify region of interest step
- 66: position marker for OCT scan step
- 68: scan and store OCT images step
- 72: crosshairs
- 74: light indicator
- 76: scan marker
- 78: scan area
- 80a, 80b: aiming light source
- 82: object plane
- 84a, 84b: images of the light source
- 86a, 86b: imaging optics for light source
- 92: polarizing beam splitter
- 94: illumination combiner
- 96: dichroic combiner
- O: optical axis

## Claims

1. An apparatus for obtaining images of a tissue comprising:
a) at least one image sensor to obtain fluorescence image and polarized reflectance images;
b) an imaging lens to image the tissue to the image sensor;
c) a first illumination means providing broadband polarized polychromatic light for obtaining the polarized reflectance image;
d) a second illumination means providing narrow-band ultraviolet light for obtaining the fluorescence light image;
e) a third illumination means providing polarized near-infrared light for obtaining the polarized reflectance image;
f) one or more polarization elements disposed in an illumination path and imaging path;
g) a long pass filter disposed in the imaging path to block the reflected narrow-band ultraviolet light from the tissue from reaching the image sensor;
h) an optical coherence tomography (OCT) imaging apparatus comprising a low coherence light source, a detector, and a coupler that splits the low coherence light into a sample path low coherence light and a reference path low coherence light;
i) a scanner for scanning the sample path low coherence light toward the tissue;
j) a processor programmed to identify a region of interest of the tissue according to either the polarized reflectance image, the fluorescence light image, or both, and to store at least one parameter of OCT scanning; and
k) a control logic processor programmed to actuate the OCT imaging apparatus to obtain an OCT image over the region of interest of the tissue.

2. The apparatus of claim 1 wherein the at least one parameter of OCT scanning includes the location and size of the region of interest, scanning mode, scanning direction, scanning step size and OCT data.

3. The apparatus of claim 1 further comprising a dichroic element disposed between the tissue and the image sensor to direct the broadband polychromatic light, the ultraviolet light, and the low coherence light toward the tissue; and direct the reflected polychromatic light and fluorescence light from the tissue to the image sensor, and the reflected low coherence light from the tissue to the detector.

4. The apparatus of claim 1 further comprising an illumination combiner disposed in the illumination path to direct the broadband polychromatic light and the narrow- band ultraviolet light along a common illumination path to illuminate the tissue.

5. The apparatus of claim 1 further comprising a folding mirror.

6. The apparatus of claim 1 wherein the wavelength of the light source in the second illumination means is greater than 350nm and less than 450nm.

7. The apparatus of claim 1 wherein the wavelength of the light source in the third illumination means is greater than 700nm.

8. The apparatus of claim 1 wherein the polarization element is polarization beam splitter.

9. The apparatus of claim 1 wherein the polarization axis of the polarization element is an electronically controllable.

10. The apparatus of claim 1 further comprising a scanning lens for obtaining an OCT image.

11. The apparatus of claim 1 wherein the scanner is a galvo scanner.

12. A method for obtaining images of a tissue comprising:
a) directing broadband polarized polychromatic light onto the tissue surface for obtaining a polarized reflectance image of the tissue;
b) directing narrow-band ultraviolet light onto the tissue surface for obtaining a fluorescence image of the tissue;
c) directing near infrared light onto the tissue surface for obtaining reflectance images with orthogonal polarization states;
d) displaying the polarized reflectance images and fluorescence image of the tissue on a display unit;
e) identifying the region of interest on the tissue from the reflectance and fluorescence images;
f) scanning the suspicious area and obtaining OCT image; and
g) storing at least one parameter of the region of interest and OCT scanning.

13. The method of claim 12 wherein the at least one parameter of OCT scanning includes the location and size of the region of interest, scanning mode, scanning direction, scanning step size and OCT data.

14. The method of Claim 12 further comprising:
directing first and second aiming lights on the tissue; and
moving toward or away from the tissue until the directed light is in right position.

15. The method of claim 14 wherein the at least one parameter of OCT scanning includes the location and size of the region of interest, scanning mode, scanning direction, scanning step size and OCT data.
